# EUROPÄISCHE PATENTSCHRIFT

(11) **EP 1 579 028 B1**
(45) Veröffentlichungstag und Bekanntmachung des Hinweises auf die Patenterteilung: **12.03.2008**
(21) Anmeldenummer: 02796576.3
(22) Anmeldetag: 06.12.2002
(51) Int. Cl.: C23C 18/00, C23C 18/12, C23C 26/00, A61L 27/32

(54) **VERFAHREN ZUR BESCHICHTUNG EINES SUBSTRATES MIT CALCIUMPHOSPHAT**
METHOD FOR COATING A SUBSTRATE WITH CALCIUM PHOSPHATE
PROCEDE POUR RECOUVRIR UN SUBSTRAT DE PHOSPHATE DE CALCIUM

(30) Priorität: 15.12.2001 DE 10161827
(43) Veröffentlichungstag der Anmeldung: 28.09.2005
(73) Patentinhaber: Dot Gmbh, D-18059 Rostock (DE)
(72) Erfinder: BECKER, Petra, 18057 Rostock (DE); BUHRMEISTER, Mischa, 18059 Rostock (DE); NEUMANN, Hans-Georg, 18146 Rostock (DE); TELLER, Marianne, 18276 Mistorf (DE)
(74) Vertreter: Garrels, Sabine
(86) Internationale Anmeldenummer: PCT/EP2002/013819
(87) Internationale Veröffentlichungsnummer: WO 2003/052164

(56) Entgegenhaltungen:
- EP-A- 0 322 250
- EP-A- 0 347 028
- US-A- 5 849 569
- US-B1- 6 323 146

## Beschreibung

### Technisches Gebiet

Die Erfindung betrifft ein Verfahren zur Beschichtung eines porösen Substrates mit einer Calciumphosphatschicht.

### Stand der Technik

Zur Beschichtung von Substraten insbesondere mit einer bioaktiven Calciumphosphatschicht, z.B. Hydroxylapatit, wird das Plasma-SprayVerfahren verwendet. Dieses Verfahren ist jedoch nicht geeignet, wenn das Substrat aus kleinen Partikeln besteht oder nicht hitzebeständig ist. Die elektrochemische Abscheidung von Calciumphosphat ist ebenfalls für Partikel nicht anwendbar, selbst wenn die Partikel elektrisch leitend sind.

In der US-PS 6,153,266 wird ein Verfahren zur Herstellung einer Calciumphosphatschicht beschrieben, bei der in einem ersten Schritt das Substrat in ein phosphathaltiges Bad getaucht wird, das kein Calcium enthält. Anschließend wird das Substrat der Lösung entnommen, getrocknet und in eine zweite Lösung getaucht, die einen ph-Wert von 8 aufweist und Calcium enthält. In dieser Lösung bildet sich an der Oberfläche des Substrates Calciumphosphat. Nachteilig bei diesem Verfahren ist zunächst, dass die Beschichtung mit 2 Verfahrensschritten durchgeführt wird. Ein weiterer Nachteil besteht darin, dass es mit dem 2-Schritt-Verfahren nicht möglich ist, die Schichtdicke im gewünschten Umfang herzustellen. Für dicke Schichten sieht deshalb das bekannte Verfahren noch einen weiteren Verfahrensschritt vor, bei dem das mit der Calciumphosphatschicht versehene Substrat in eine Calciumphosphatlösung getaucht wird.

Alle in EP-A1-0 322 250 aufgeführten Beispiele werden durch Lufttrocknung bzw. Sinterung vom anhaftendem Wasser befreit. Es wird in jedem Fall unter Normaldruck gearbeitet. In einem Fall wird die Beschichtung durch Zentrifugation aufgebracht.

In US-B1-6 232 146 wird ein Calciumphosphat-Komposit hergestellt, das zusätzlich Silicium oder Titan als elementaren Bestandteil des Sols enthalten kann. Diese Komposite werden durch Zentrifugation von der Herstellungslösung abgetrennt und dann thermisch weiterbehandelt. Eine Porosität wird u.a. durch Ausheizen organischer Zusätze erreicht.

In EP-A2-0 347 028 wird die Calciumphosphatverbindung durch Verkneten oder Mischen der Phosphorsäurekomponente mit einer Calciumkomponente unter Zugabe von Natriumhydroxid erzeugt. Es wird in jedem Fall unter Normaldruck gearbeitet. Die fertige Mischung kann subcutan oder auch intramuskular appliziert werden.

Nachteil dieser Lösungen ist es, dass unter Normaldruck gearbeitet wird und dadurch in den Poren befindliche Gase verbleiben können bzw. ein Haften der Beschichtungen erschwert wird.

### Darstellung der Erfindung

Der Erfindung liegt die Aufgabe zugrunde, die Nachteile der bekannten Verfahren zu beseitigen und in einem einfachen Verfahren eine Calciumphosphatschicht herzustellen, die eine gewünschte Schichtdicke aufweist.

Erfindungsgemäß wird diese Aufgabe dadurch gelöst, dass folgende Schritte durchlaufen werden: Eintragen des Substrates in ein Calciumphosphat-Gel, Entfernen der am Substrat befindlichen Luft durch Anlegen eines Unterdrucks, Belüftung des Gemisches aus Substrat und Gel, Trocknen des mit dem Gel beschichteten Substrates und Abtrennung von Calciumphosphat-Partikeln vom beschichteten Substrat oder Eintragen des Substrates in ein Calciumphosphat enthaltendes SiO₂-Gel oder -Kolloid, Entfernen von Luft und Lösungsmittel durch Anlegen eines Unterdrucks und Aufarbeitung wie vorstehend beschrieben.

Vorteilhafte Ausgestaltungen der Erfindung werden in den Unteransprüchen beschrieben.

Der Vorteil des erfindungsgemäßen Verfahrens besteht darin, dass es möglich ist, ein Substrat in einem Schritt mit einer Calciumphosphatschicht zu versehen. Da die Schichtdicke von der Konzentration des Calciumphosphat-Gels bzw. der kolloiden Lösung abhängt, kann durch Einstellung der Konzentration eine gewünschte Schichtdicke hergestellt werden. Das Verfahren ist für die Beschichtung großflächiger Substrate und von Partikeln geeignet. Es ist unabhängig davon, aus welchem Material das Substrat besteht. Die Haftfestigkeit der Schicht auf dem Substrat wird dadurch erhöht , dass durch Anlegen eines Unterdruckes das an dem Substrat befindliche Gas (Luft) entfernt wird. Dieses Verfahren ist besonders vorteilhaft, wenn das Substrat Poren aufweist. Es wird dann die Luft aus den Poren entfernt und das Calciumphosphat-Gel kann in die Poren eindringen.

Ein weiterer Vorteil ist darin zu sehen, dass es möglich ist, durch eine geeignete Zusammensetzung des Gels die Schicht aus verschiedenen Calciumphosphatmodifikationen herzustellen. Dies ist besonders von Bedeutung für eine bioaktive Schicht. Es ist bekannt, dass der Grad der Bioaktivität einer Schicht mit ihrer Instabilität in physiologischer Umgebung wächst. Calciumphosphate mit einer im Vergleich zum Hydroxylapatit sehr viel höheren Löslichkeit wie Bruschit und Monetit haben speziell für die Einheilungsphase in den Knochen eine besondere Bedeutung. Sollen die beschichteten Partikel mit der Zielsetzung verwendet werden, den Verbund des Substrates mit Knochengewebe zu verbessern, besteht das Gel aus Bruschit und/oder Monetit oder aus einem Gemisch aus Bruschit und/oder Monetit und Hydroxylapatit. Das Gel kann aber auch aus einem Gemisch aus Silica-Gel und Calciumphosphat bestehen. Es kann ferner Füllstoffe wie Calciumsulfat oder Faktoren enthalten, die knocheninduzierend wirken. Es ist auch möglich, in die Schicht Arzneimittel wie Antibiotika, das Wachstum des Knochens beschleunigende oder den Abbau des Knochens hemmende Mittel einzubringen, indem sie z.B. mit in das Gel eingemischt werden.

Die mit dem erfindungsgemäßen Verfahren beschichteten Substrate sind besonders geeignet als Knochenersatzmaterial zur Auffüllung von Knochendefekten oder zur Ausfüllung eines Spaltes zwischen einem Implantat und einem Knochen, in den das Implantat eingesetzt wurde. Für diese Zwecke werden bevorzugt beschichtete Substratpartikel verwendet, die biokompatibel sind. Die Partikel können auch dazu dienen, das Implantat im Knochen zu verankern.

### Weg(e) zur Ausführung der Erfindung

Die Erfindung wird im folgenden an Hand von Beispielen näher erläutert, ohne darauf beschränkt zu sein.

Beispiel 1:

In eine Calciumhydroxid-Lösung wird unter ständigem Rühren eine Phosphatsäurelösung eingetragen. Der sich bildende feine Niederschlag wird durch Zentrifugation aufkonzentriert und von er überstehenden klaren Lösung abgetrennt. In das so erhaltene Calciumphosphat-Gel wird ein Titan-Granulat, das zuvor entfettet und gereinigt wurde, eingebracht und gut durchmischt. Dieses Gemisch wird in eine Unterdruckkammer gebracht. Beim Anlegen eines geeigneten Unterdruckes wird die Luft aus den Poren des Titan-Granulates entfernt. Beim anschließenden Belüften dringt das Calciumphosphat-Gel in die Poren ein. Das beschichtete Granulat wird getrocknet, vereinzelt und durch Siebung von abgefallenen Calciumphosphatpartikeln getrennt. Die Schichtdicke des Calciumphosphates ist variabel und kann über die -Konzentration des hergestellten Gels eingestellt werden.

Beispiel 2:

Ein beliebiges Calciumphosphatpulver wird mit einem Silica-Gel gemischt. In dieses Gel wird ein entfettetes und gereinigtes Titan-Granulat eingebracht und gut durchmischt. Wie im Beispiel 1 wird die Luft aus den Poren des Titan-Granulates entfernt, um beim Belüften das Eindringen des Calciumphosphat-Silica-Gels in die Poren zu ermöglichen. Das beschichtete Granulat wird getrocknet, vereinzelt und mittels eines Luftstromes von ausgefallenen Calciumphosphat-Silica-Partikeln getrennt. Die Schichtdicke des Calciumphosphat-Silikates kann über die Konzentration des Gels eingestellt werden.

## Patentansprüche

1. Verfahren zur Beschichtung eines porösen Substrates mit einer Calciumphosphatschicht, **dadurch gekennzeichnet, dass** folgende Schritte durchlaufen werden:
a) Eintragen des Substrates in ein Calciumphosphat-Gel, das mindestens eine oder mehrere Calciumphosphat-Phase(n) enthält,
b) Entfernen der am Substrat befindlichen Luft durch Anlegen eines Unterdrucks,
c) Belüftung des Gemisches aus Substrat und Gel,
d) Trocknen des mit dem Gel beschichteten Substrates,
e) Abtrennung von Ca!ciumphosphat-Partikeln vom beschichteten Substrat.

2. Verfahren zur Beschichtung eines porösen Substrates mit einer Calciumphophatschicht, **dadurch gekennzeichnet, dass** folgende Schritte durchlaufen werden
a) Eintragen des Substrates in ein Calciumphosphat enthaltendes SiO₂-Gel oder -Kolloid,
b) Entfernen der Luft durch Anlegen eines Unterdrucks,
c) Belüftung des Gemisches aus Substrat und Gel,
d) Trocknen des mit dem Gel beschichteten Substrates,
e) Abtrennung von Ca!ciumphosphat-Partikeln vom beschichteten Substrat.

3. Verfahren nach Anspruch 1 und 2, **dadurch gekennzeichnet, dass** die Schichtdicke durch Veränderung der Konzentration des Calciumphosphat-Gels bzw. der kolloiden Lösung bestimmt wird.

4. Verfahren nach Anspruch 1, **dadurch gekennzeichnet, dass** das Calciumphosphat-Gel aus Bruschit und/oder Monetit oder einem Gemisch aus Bruschit und/oder Monetit und Hydroxylapatit besteht.

5. Verfahren nach Anspruch 1, **dadurch gekennzeichnet, dass** das Gel aus einem Gemisch aus Silica-Gel und Calciumphosphat besteht.

6. Verfahren nach Anspruch 2, **dadurch gekennzeichnet, dass** die kolloide Lösung Hydroxylapatit und/oder β-Tricalciumphosphat enthält.

7. Verfahren nach Anspruch 1 oder 2 und einem oder mehreren der vorhergehenden Ansprüche, **dadurch gekennzeichnet, dass** das Calciumphosphat-Gel bzw. die kolloide Lösung knocheninduzierende Faktoren enthält.

8. Verfahren nach Anspruch 1 oder 2 und einem oder mehreren der vorhergehenden Ansprüche, **dadurch gekennzeichnet, dass** das Calciumphosphat-Gel bzw. die kolloide Lösung Arzneimittel enthält.

## Claims

1. A method for coating a porous substrate with a calcium phosphate layer, **characterized in that** the following steps are to be passed through:
a) Feeding said substrate into a calcium phosphate gel containing at least one calcium phosphate phase or more calcium phosphate phases;
b) Removing of air contained on said substrate by applying a negative pressure;
c) Ventilating the mixture made of said substrate and said gel;
d) Drying said substrate coated with said gel;
e) Separation of calcium phosphate particles from said coated substrate.

2. A method for coating a porous substrate with a calcium phosphate layer, **characterized in that** the following steps are to be passed through:
a) Feeding said substrate into a SiO₂ gel or SiO₂ colloid containing calcium phosphate;
b) Removing of air by applying a negative pressure;
c) Ventilating said mixture made of said substrate and said gel;
d) Drying said substrate coated with said gel;
e) Separation of calcium phosphate particles from said coated substrate;

3. A method according to claim 1 and 2, **characterized in that** the layer thickness is determined by modification of the concentration of said calcium phosphate gel and the colloidal solution, respectively.

4. A method according to claim 1, **characterized in that** said calcium phosphate gel consists of brushite and/or monetite, or of a mixture of said brushite and/or said monetite and hydroxylapatite.

5. A method according to claim 1, **characterized in that** said gel consists of a mixture of silica gel and said calcium phosphate.

6. A method according to claim 2, **characterized in that** said colloidal solution contains hydroxylapatite and/or β-tricalcium phosphate.

7. A method according to claim 1 or 2, and any one or several of the preceding claims, **characterized in that** said calcium phosphate gel and said colloidal solution, respectively, contain bone-inducing factors.

8. A method according to claim 1 or 2, and any one or several of the preceding claims, **characterized in that** said calcium phosphate gel and said colloidal solution, respectively, contain pharmaceutical preparations.

## Revendications

1. Procédé pour la déposition d'un substrat poreux avec une couche des phosphates de calcium, **caractérisé en ce que** les pas suivants seront parcourus:
a) rapporter ledit substrat dans un gel de phosphate de calcium, lequel contient au moins une phase ou plusieurs phases de ledit phosphate de calcium;
b) enlever de l'air se trouvant à ledit substrat au moyen d'établir d'une dépression ;
c) ventilation de mélange de ledit substrat et ledit gel;
d) dessécher ledit substrat déposé avec ledit gel;
e) séparation de particules de ledit phosphate de calcium en ledit substrat déposé;

2. Procédé pour la déposition d'un substrat poreux avec une couche des phosphates de calcium, **caractérisé en ce que** les pas suivants seront parcourus:
a) rapporter ledit substrat dans un gel ou colloide de SiO₂ contenant de phosphate de calcium;
b) enlever de l'air au moyen d'établir d'une dépression;
c) ventilation ledit mélange de ledit substrat et ledit gel;
d) dessécher ledit substrat déposé avec ledit gel;
e) séparation de particules de ledit phosphate de calcium de ledit substrat déposé.

3. Procédé selon la revendication 1 et 2, **caractérisé en ce que** l'épaisseur de la couche est déterminé au moyen d'une variation de la concentration de ledit gel des phosphates de calcium ou bien la solution colloïdale.

4. Procédé selon la revendication 1, **caractérisé en ce que** ledit gel des phosphates de calcium consiste en brushite et/ou monetite ou en mélange de brushite et/ou monetite et hydroxylapatite.

5. Procédé selon la revendication 1, **caractérisé en ce que** ledit gel consiste en mélange de silicagel et ledit phosphate de calcium.

6. Procédé selon la revendication 2, **caractérisé en ce que** ladite solution colloïdale contient de hydroxylapatite et/ou de β-tricalciumphosphate.

7. Procédé selon la revendication 1 ou 2 et selon l'une quelconque ou plusieurs des revendications précédentes, **caractérisé en ce que** ledit gel des phosphates de calcium ou bien ladite solution colloïdale contient de facteurs induisant des os.

8. Procédé selon la revendication 1 ou 2 et selon l'une quelconque ou plusieurs des revendications précédentes, **caractérisé en ce que** ledit gel des phosphates de calcium ou bien ladite solution colloïdale contient de médicaments.
